# EUROPÄISCHE PATENTSCHRIFT

(11) **EP 1 254 653 B1**
(45) Veröffentlichungstag und Bekanntmachung des Hinweises auf die Patenterteilung: **08.09.2004**
(21) Anmeldenummer: 01110624.2
(22) Anmeldetag: 30.04.2001
(51) Int. Cl.: A61K 7/50

(54) **Verwendung von kationischen Zubereitungen**
Use of cationic compositions
Utilisation de compositions cationiques

(43) Veröffentlichungstag der Anmeldung: 06.11.2002
(73) Patentinhaber: Cognis Iberia, S.L., 08755 Castellbisbal, (Barcelona) (ES)
(72) Erfinder: Prat Queralt, Esther, 08328 Alella (ES); Amela Conesa, Christina, 08029 Cerdanyola del Vallés (ES)
(74) Vertreter: Fabry, Bernd, Dr.

(56) Entgegenhaltungen:
- EP-A- 0 786 250
- EP-A- 0 875 500
- EP-A- 0 885 607
- DE-A- 19 754 283
- DE-A- 19 962 874
- DE-C- 19 743 687

## Beschreibung

### Gebiet der Erfindung

Die Erfindung befindet sich auf dem Gebiet der Kosmetik und betrifft die Verwendung von Mischungen aus Esterquats und Fettsäureamidoaminen zur Herstellung von kosmetischen Zubereitungen, speziell solchen für die Haarpflege.

### Stand der Technik

Kationische Tenside besitzen im allgemeinen die Eigenschaft auf die negativ geladenen Keratinfasern aufzuziehen. Dabei bewirken sie einen abstoßenden Effekt zwischen den Filamenten, was dazu führt, dass sich die Haare leichter kämmen lassen, weniger stark elektrisch aufgeladen werden und sich im ganzen weicher anfühlen. Gelegentlich wird auch der Glanz verbessert. Obschon es sich hierbei wie schon gesagt um eine mehr oder weniger generische Eigenschaft der Kationtenside handelt, sind die zu erzielenden Effekte natürlich stark von der Struktur der eingesetzten Spezies oder deren erfolgreicher Abmischung abhängig. Nachdem es denkgesetzlich eine praktisch unbegrenzte Anzahl von kationisch geladenen oberflächenaktiven Substanzen gibt, bedarf es gar nicht der Möglichkeit diese untereinander zu permutieren, um ein ungeheuer weites Feld an Möglichkeiten zu eröffnen, innerhalb dessen jederzeit neue Zubereitungen mit interessanten oder sogar speziell zugeschnittenen Eigenschaften aufgefunden werden können.

Bei der Entwicklung moderner kosmetischer Zubereitungen, speziell von Haarpflegemitteln, geht man über das bekannte Prinzip "two-in-one" inzwischen weit hinaus. Längst sind "three-in-one" Lösungen auf dem Markt und der Trend geht in eine Richtung, die man gerne als "all-in-one" bezeichnen möchte. Anders ausgedrückt wird die Entwicklung von Mitteln gewünscht, die einerseits möglichst viele, teilweise sehr unterschiedliche Wirkungen hervorrufen, andererseits jedoch möglichst wenige Bestandteile aufweisen, um die Formulierungen einfach in der Herstellung und für den Kunden bezahlbar zu machen. Es liegt auf der Hand, dass solche Zubereitungen nicht dadurch erhältlich sind, indem man gängige Handelsprodukte einfach abmischt - andernfalls wären solche Produkte längst auf dem Markt vertreten. Vielmehr geht es darum, aus der Gruppe der bekannten Kationtenside solche Vertreter zu identifizieren, die falls möglich alleine, vorzugsweise aber in synergistischer Abmischung mit anderen Stoffen das gewünschte Anforderungsprofil aufweisen.

Im vorliegenden Fall hat die Aufgabe der Erfindung darin bestanden, transparente kosmetische Zubereitungen, speziell zur Haarpflege und insbesondere für die Haarkonditionierung zur Verfügung zu stellen, die lager- und temperaturstabile Einarbeitung sowohl von Siliconölen als auch Antischuppenwirkstoffen erlauben. Gleichzeitig sollten Weichgriff und antistatische Ausrüstung der Haare wenigstens das Niveau des Stands der Technik erreichen.

### Beschreibung der Erfindung

Gegenstand der Erfindung ist die Verwendung von Mischungen, enthaltend deren Acylreste sich von gemischen von Monocarbonsäuren und Dicarbonsäuren ableiten, und
(a) Esterquats
(b) Fettsäureamidoamine
zur Herstellung von kosmetischen Zubereitungen.

Überraschenderweise wurde gefunden, dass Abmischungen von (a) Esterquats, deren Acylreste Mischungen von Mono- und Dicarbonsäuren darstellen, und (b) Fettsäureamidoaminen das gewünschte Anforderungsprofil erfüllen. Die Zubereitungen sind bei der Herstellung transparent und trüben auch nicht bei Lagerung ein. Sowohl Siliconöle als auch Antischuppenwirkstoffe lassen sich auch in höheren Konzentrationen stabil einarbeiten und werden selbst bei Lagerung über 4 Wochen bei 40 °C nicht wieder ausgeschieden. Die behandelten Haare zeigen einen ausgezeichneten Weichgriff und eine verminderte Kämmarbeit, wobei der Stand der Technik in einigen Fällen sogar übertroffen wird.

### Esterquats

Unter der Bezeichnung "Esterquats" (Komponente a) werden im allgemeinen quaternierte Fettsäurealkanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Aus der Deutschen Patentschrift **DE 4308794 C1** (Henkel) ist überdies ein Verfahren zur Herstellung fester Esterquats bekannt, bei dem man die Quaternierung von Triethanolaminestern in Gegenwart von geeigneten Dispergatoren, vorzugsweise Fettalkoholen, durchführt. Übersichten zu diesem Thema sind beispielsweise von R.Puchta et al. in **Tens.Surf.Det., 30, 186 (1993),** M.Brock in **Tens.Surf.Det. 30, 394 (1993),** R.Lagerman et al. in **J.Am. Oil.Chem.Soc., 71, 97 (1994)** sowie I.Shapiro in **Cosm.Toil. 109, 77 (1994)** erschienen.

Die quaternierten Fettsäuretriethanolaminestersalze folgen beispielsweise der Formel **(I),** 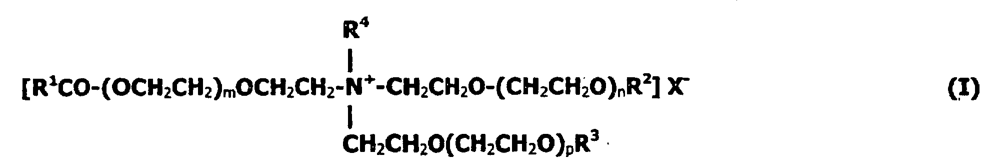 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht. Typische Beispiele für Esterquats, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielsweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt. Zur Herstellung der quaternierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von C₈-C₁₀-Vorlauffettsäure, Talgfettsäure, Palmfettsäure, Kokosfettsäure, Ölsäure, Ricinolsäure sowie deren Härtungsprodukten und Gemischen (Iodzahl 0 bis 40) ab. Aus anwendungstechnischer Sicht haben sich quaternierte Fettsäuretriethanolaminestersalze der Formel **(I)** als besonders vorteilhaft erwiesen, in der R¹CO für seinen Acylrest mit 16 bis 18 Kohlenstoffatomen, R² für R¹CO, R³ für Wasserstoff, R⁴ für eine Methylgruppe, m, n und p für 0 und X für Methylsulfat steht. Entsprechende Produkte sind unter der Marke Dehyquart® AU (Cognis Deutschland GmbH) im Handel.

Neben den quaternierten Fettsäuretriethanolaminestersalzen kommen als Esterquats ferner auch quaternierte Estersalze von Fettsäuren mit Diethanolalkylaminen der Formel **(II)** in Betracht, 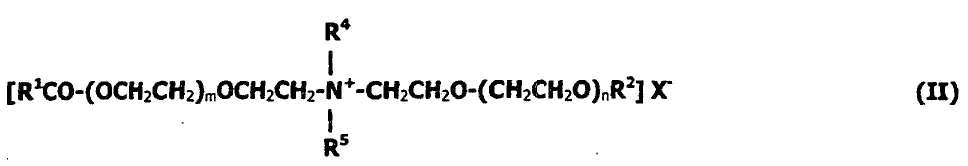 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht

Als weitere Gruppe geeigneter Esterquats sind schließlich die quaternierten Estersalze von Fettsäuren mit 1,2-Dihydroxypropyldialkylaminen der Formel **(III)** zu nennen, 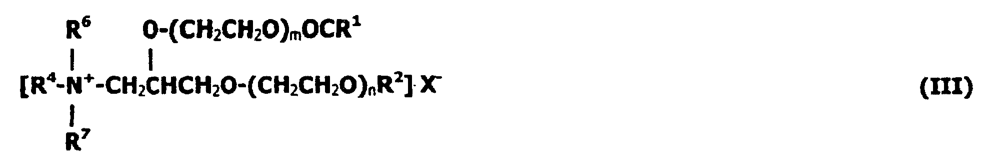 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Des weiteren kommen als Esterquats noch Stoffe in Frage, bei denen die Ester- durch eine Amidbindung ersetzt ist und die vorzugsweise basierend auf Diethylentriamin der Formel **(IV)** folgen, 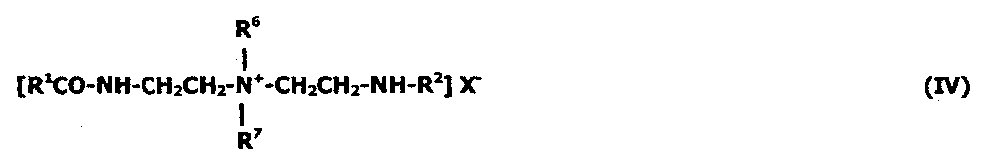 in der R¹CO für einen Acylrest mit 6 bis 22 Kohlenstoffatomen, R² für Wasserstoff oder R¹CO, R⁶ und R⁷ unabhängig voneinander für Alkylreste mit 1 bis 4 Kohlenstoffatomen und X für Halogenid, Alkylsulfat oder Alkylphosphat steht. Derartige Amidesterquats sind beispielsweise unter der Marke Incroquat® (Croda) im Markt erhältlich.

Schließlich kommen als Esterquats auch Stoffe in Frage, die auf Basis von ethoxyliertem Ricinusöl oder dessen Härtungsprodukten erhältlich sind und vorzugsweise der Formel **(V)** folgen, 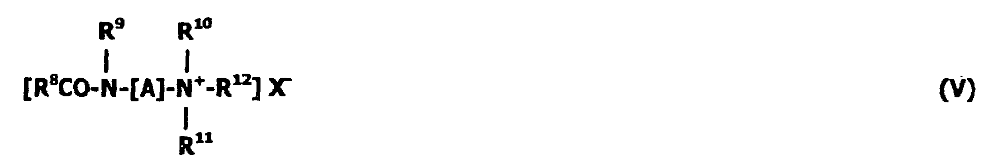 in der R⁸CO für einen gesättigten und/oder ungesättigten ethoxylierten Hydroxyacylrest mit 16 bis 22, vorzugsweise 18 Kohlenstoffatomen sowie 1 bis 50 Oxyethyleneinheiten, A für einen linearen oder verzweigten Alkylenrest mit 1 bis 6 Kohlenstoffatomen, R⁹, R¹⁰ und R¹¹ unabhängig voneinander für Wasserstoff oder eine Alkylgruppe mit 1 bis 4 Kohlenstoffatomen, R¹² für einen Alkylrest mit 1 bis 4 Kohlenstoffatomen oder einen Benzylrest und X für Halogen, Alkylsulfat oder Alkylphosphat steht.

Hinsichtlich der Auswahl der bevorzugten Fettsäuren und des optimalen Veresterungsgrades gelten die für **(I)** genannten Beispiele auch für die Esterquats der Formeln **(II)** bis **(V)**.

Zur Herstellung der Esterquats der Formeln **(I)** bis **(V)** kann sowohl von Fettsäuren als auch den entsprechenden Triglyceriden ausgegangen werden. Ein solches Verfahren, das stellvertretend für den entsprechenden Stand der Technik genannt werden soll, wird in der europäischen Patentschrift **EP 0750606 B1** (Cognis) vorgeschlagen. Ebenfalls ist es möglich, die Kondensation der Alkanolamine mit den Fettsäuren in Gegenwart definierter Mengen an Dicarbonsäuren, wie z.B. Oxalsäure, Malonsäure, Bernsteinsäure, Maleinsäure, Fumarsäure, Glutarsäure, Adipinsäure, Sorbinsäure, Pimelinsäure, Azelainsäure, Sebacinsäure und/oder Dodecandisäure durchzuführen. Auf diese Weise kommt es zur einer partiell oligomeren Struktur der Esterquats, was sich insbesondere bei Mitverwendung von Adipinsäure auf die Klarlöslichkeit der Produkte vorteilhaft auswirken kann. Entsprechende Produkte unter der Marke Dehyquart® D 6003 (Cognis Deutschland GmbH) sind im Handel erhältlich und werden beispielsweise in der Europäischen Patentschrift **EP 0770594** B1 (Cognis) beschrieben. Üblicherweise gelangen die Esterquats in Form 50 bis 90 Gew.-%iger alkoholischer Lösungen in den Handel, die bei Bedarf problemlos mit Wasser verdünnt werden können.

### Fettsäureamidoamine

Auch die Fettsäureamidoamine, die die Komponente (b) bilden, stellen bekannte Stoffe dar, die beispielsweise durch Umsetzung von Fettsäuren mit mehrwertigen in an sich bekannter Weise erhalten werden können. Vorzugsweise werden Stoffe der Formel **(VI)** eingesetzt,

**R**^{**13**}**CO-[NH(B)]**_{**n**}**NR**^{**14**}**R**^{**15**} **(VI)**

in der R¹³ für einen linearen oder verzweigten, gesättigten oder ungesättigten Acylrest mit 6 bis 22 Kohlenstoffatomen, R¹⁴ und R¹⁵ unabhängig voneinander für Wasserstoff, einen gegebenenfalls hydroxysubstituierten Alkylrest mit 1 bis 4 Kohlenstoffatomen, B für eine lineare oder verzweigte Alkylengruppe mit 2 bis 4 Kohlenstoffatomen und n für 1 oder 2 steht. Bezüglich der Fettsäuren können sich die Amidoamine von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen ableiten, wobei Fettsäureamidoamine auf Basis von Kokosfettsäuren bevorzugt sind. Ebenfalls bevorzugt sind Verbindungen deren Amidkomponente sich von Ethylendiamin, Propylendiamin, Diethylentriamin, Dipropylentriamin, Aminoethylethanolamin und insbesondere N,N,-Dimethylaminopropylamin sowie deren Gemischen ableitet. In Summe bevorzugt sind die Fettsäureamidoamine, die die einzelnen als bevorzugt genannten Strukturmerkmale in sich vereinigen.

Es ist möglich, die Esterquats und die Fettsäureamidoamine im Gewichtsverhältnis 99,1 : 0,1 bis 10 : 90 einzusetzen. Aus anwendungstechnischen Gründen hat sich jedoch ein Verhältnis von 75 : 25 bis 25 : 75 und insbesondere 60 : 40 bis 40 : 60 als besonders vorteilhaft erwiesen, da hier synergistische Leistungsverstärkungen beobachtet werden.

### Fettalkohole und Fettalkoholpolyglycolether

In einer bevorzugten Ausführungsform der Erfindung werden die Esterquats und die Fettsäureamidoamine gemeinsam mit Fettalkoholen und/oder Fettalkoholpolyglycolethern eingesetzt. Auf diese Weise lassen sich beispielsweise besonders stabile Emulsionen und Cremes für die Haarbehandlung, speziell die Haarkonditionierung herstellen. Die Fettalkohole bzw. Fettalkoholpolyglycolether folgen vorzugsweise der Formel **(VII),**

**R**^{**16**}**O(CH**_{**2**}**CH**_{**2**}**O)**_{**m**}**H (VII)**

in der R¹⁶ für einen linearen oder verzweigten Alkyl- und/oder Alkenylrest mit 6 bis 22, vorzugsweise 12 bis 18 Kohlenstoffatomen und m für 0 oder Zahlen von 1 bis 20, vorzugsweise 10 bis 15 steht. Typische Beispiele sind Capronalkohol, Caprylalkohol, Caprinalkohol, Laurylalkohol, Myristylalkohol, Cetylalkohol, Stearylalkohol, Isostearylalkohol, Oleylolakohol, Elaidylalkohol, Petroselinylalkohol, Arachylalkohol, Gadoleylalkohol, Behenylalkohol und Erucylalkohol sowie deren technische Gemische, wie beispielsweise Kokosalkohol oder Ceterylalkohol sowie deren Ethylenoxid-Anlagerungsprodukte. Die Esterquats und Fettsäureamidoamine einerseits und die Fettalkohole bzw. Fettalkoholpolyglcolether andererseits können wiederum im Gewichtsverhältnis 90 : 10 bis 10 : 90 eingesetzt werden, wobei hier ein Verhältnis von 35 : 65 bis 50 : 50 bevorzugt ist.

### Siliconöle und Antischuppenwirkstoffe

Wie schon eingangs erwähnt, besteht ein besonderes Anliegen der Erfindung darin, Siliconöle und Antischuppenwirkstoffe, die sich üblicherweise nur schwer in Emulsionen einarbeiten lassen und die Tendenz zeigen, sich bei längerer Lagerung zumal bei Temperatureinfluss wieder abzuscheiden, stabil einzuarbeiten.

Geeignete Siliconöle sind beispielsweise Dimethylpolysiloxane, Methylphenylpolysiloxane, cyclische Silicone sowie amino-, fettsäure-, alkohol-, polyether-, epoxy-, fluor-, glykosidund/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).** Die Siliconöle können in Mengen von 1 bis 20, vorzugsweise 5 bis 15 Gew.-% - bezogen auf die Zubereitungen - eingesetzt werden.

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfelteer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage. Die Antischuppenwirkstoffe können in Mengen von 0,1 bis 2, vorzugsweise 0,5 bis 1,5 Gew.-% - bezogen auf die Zubereitungen - eingesetzt werden.

### Gewerbliche Anwendbarkeit

Ein weiterer Gegenstand der Erfindung betrifft die Verwendung der Gemische der Komponenten (a) und (b) zur Herstellung von Haarpflegemitteln, speziell von Haarkonditioniermitteln, vorzugsweise solchen in Emulsionsform, bei denen es sich insbesondere um Haarcremes handelt, welche die Gemische jeweils in Mengen von 1 bis 30, vorzugsweise 2 bis 20 und insbesondere 5 bis 15 Gew.-% - bezogen auf die Zubereitungen - enthalten können. Ein letzter Gegenstand der Erfindung betrifft die Verwendung der Gemische der Komponenten (a) und (b) zur Herstellung von Hautpflegemitteln, insbesondere von solchen in Emulsionsform.

### Kosmetische Zubereitungen

Diese kosmetischen Zubereitungen, zu deren Herstellung die kationischen Gemische dienen, können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Fette, Wachse, Lecithine, Phospholipide, biogene Wirkstoffe, UV-Lichtschutzfaktoren, Antioxidantien, Deodorantien, Antitranspirantien, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, I-sostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. **DE 19756377 A1**), insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestem mit Polyolen und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
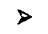 Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
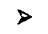 Alkyl- und/oder Alkenyloligoglykoside mit 8 bis 22 Kohlenstoffatomen im Alk(en)ylrest und deren ethoxylierte Analoga;
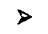 Anlagerungsprodukte von 1 bis 15 Mol Ethylenoxid an Ricinusöl und/oder gehärtetes Ricinusöl;
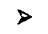 Anlagerungsprodukte von 15 bis 60 Mol Ethylenoxid an Ricinusöl und/oder gehärtetes Ricinusöl;
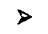 Partialester von Glycerin und/oder Sorbitan mit ungesättigten, linearen oder gesättigten, verzweigtem Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
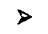 Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;.
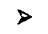 Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
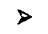 Mono-, Di- und Trialkylphosphate sowie Mono-, Di- und/oder Tri-PEG-alkylphosphate und deren Salze;
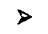 Wollwachsalkohole;
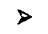 Polysiloxan-Polyalkyl-Polyether-Copolymere bzw. entsprechende Derivate;
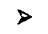 Block-Copolymere z.B. Polyethylenglycol-30 Dipolyhydroxystearate;
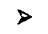 Polymeremulgatoren, z.B. Pemulen-Typen (TR-1,TR-2) von Goodrich;
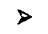 Polyalkylenglycole sowie
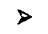 Glycerincarbonat.

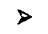 Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

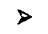 Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

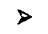 Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

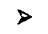 Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

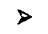 Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

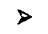 Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

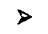 Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylatund eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropion-säuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohtenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminiumund/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar@ C-17, Jaguar@ C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in **Cosm.Toil. 108, 95 (1993)** aufgeführt.

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
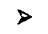 3-Benzylidencampher bzw. 3-Benzylidennorcampher und dessen Derivate, z.B. 3-(4-Methylbenzyliden)campher wie in der **EP 0693471 B1** beschrieben;
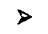 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
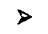 Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
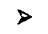 Ester der Salicylsäure, vorzugsweise Salicylsäure-2-ethylhexylester, Salicylsäure-4-isopropylbenzylester, Salicylsäurehomomenthylester;
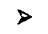 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
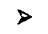 Ester der Benzalmalonsäure, vorzugsweise 4-Methoxybenzmalonsäuredi-2-ethylhexylester;
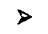 Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
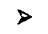 Propan-1,3-dione, wie z.B. 1-(4-tert.Butylphenyl)-3-(4'methoxyphenyl)propan-1,3-dion;
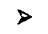 Ketotricyclo(5.2.1.0)decan-Derivate, wie in der **EP 0694521 B1** beschrieben.

Als wasserlösliche Substanzen kommen in Frage:
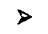 2-Phenylbenzimidazol-5-sulfonsäure und deren Alkali-, Erdalkali-, Ammonium-, Alkylammonium-, Alkanolammonium- und Glucammoniumsalze;
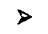 Sulfonsäurederivate von Benzophenonen, vorzugsweise 2-Hydroxy-4-methoxybenzophenon-5-sulfonsäure und ihre Salze;
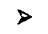 Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'methoxydibenzoylmethan (Parsol® 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Besonders günstige Kombinationen bestehen aus den Derivate des Benzoylmethans" z.B. 4-tert.-Butyl-4'-methoxydibenzoylmethan (Parsol® 1789) und 2-Cyano-3,3-phenylzimtsäure-2-ethylhexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** sowie **Parf.Kosm. 3, 11 (1999)** zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

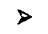 Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

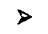 Enzvminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

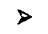 Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

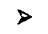 Antitranspirantien

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
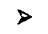 adstringierende Wirkstoffe,
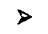 Ölkomponenten,
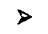 nichtionische Emulgatoren,
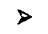 Coemulgatoren,
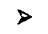 Konsistenzgeber,
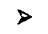 Hilfsstoffe wie z. B. Verdicker oder Komplexierungsmittel und/oder
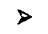 nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkoniumtetrachlorohydrat, Aluminium-Zirkoniumpentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
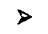 entzündungshemmende, hautschützende oder wohlriechende ätherische Öle,
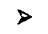 synthetische hautschützende Wirkstoffe und/oder
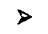 öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

### Insekten-Repellentien

Als Insekten-Repellentien kommen N,N-Diethyl-m-toluamid, 1,2-Pentandiol oder Ethyl Butylacetylaminopropionate in Frage

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
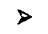 Glycerin;
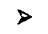 Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
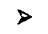 technische Oligoglyceringemische mit einem Eigenkondensationsgrad von 1,5 bis 10 wie etwa technische Diglyceringemische mit einem Diglyceringehalt von 40 bis 50 Gew.-%;
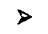 Methyolverbindungen, wie insbesondere Trimethylolethan, Trimethylolpropan, Trimethylolbutan, Pentaerythrit und Dipentaerythrit;
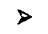 Niedrigalkylglucoside, insbesondere solche mit 1 bis 8 Kohlenstoffen im Alkylrest, wie beispielsweise Methyl- und Butylglucosid;
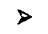 Zuckeralkohole mit 5 bis 12 Kohlenstoffatomen, wie beispielsweise Sorbit oder Mannit,
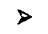 Zucker mit 5 bis 12 Kohlenstoffatomen, wie beispielsweise Glucose oder Saccharose;
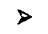 Aminozucker, wie beispielsweise Glucamin;
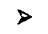 Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Y-lang-Ylang), Stengeln und Blättern (Geranium, Patchouli, Petitgrain), Früchten (Anis; Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcydohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt. Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergieichen in Frage.

### Farbstoffe

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

Verschiedene siliconöl- bzw. wirkstoffhaltige Emulsionen wurden auf ihre Stabilität unter.sucht. Bei den eingesetzten Esterquats handelte es sich ausschließlich um methylquaternierte Triethanolaminester, die als Methosulfate vorlagen und sich lediglich in ihren Acylkomponenten unterschieden, nämlich :

| | |
|---|---|
| EQ1 | Palmfettsäure (Dehyquart® AU 56) |
| EQ2 | Ölsäure und Adipinsäure (60:4, Dehyquart® AU 04) |
| EQ3 | Kokosfettsäure und Adipinsäure (60:40, Dehyquart® AU 93) |
| EQ4 | Caprylsäure und Adipinsäure (70:30, Dehyquart® ABIO 8) |
| EQ5 | Ricinolsäure+18EO |

Bei den eingesetzten Fettsäureamidoaminen handelte es sich ausschließlich um Kondensationsprodukte von Kokosfettsäure, die sich lediglich in ihren Amidkomponenten unterschieden, nämlich :

| | |
|---|---|
| FSAA1 | Ethylendiamin |
| FSAA2 | Diethylentriamin |
| FSAA3 | Aminoethylethanolamin |
| FSAA4 | N,N-Dimethylaminopropylamin |

Die Ergebnisse der Stabilitätsuntersuchungen sind in Tabelle 1 zusammengefasst. Dabei bedeutet (+++) = keine Veränderung, (++) = leichte Trübung, (+) = geringfügige Ausscheidungen, (-) = deutliche Ausscheidungen und (--) vollständige Separierung. Die Beispiele 1 bis 7 sind erfindungsgemäß, die Beispiele V1 bis V5 dienen zum Vergleich.

In Tabelle 2 finden sich einige Rezepturbeispiele.

**Tabelle 1**

| **Stabilität von Haarpflegeemulsionen (Mengenangaben als Gew.-%)** | | | | | | | | | | | | |
|---|---|---|---|---|---|---|---|---|---|---|---|---|
| | **1** | **2** | **3** | **4** | **5** | **6** | **7** | **V1** | **V2** | **V3** | **V4** | **V5** |
| EQ1 | 3 | - | - | - | - | - | 3 | 5 | - | - | - | - |
| EQ2 | - | 3 | 4 | - | - | - | - | - | 5 | - | - | - |
| EQ3 | - | - | - | 3 | - | - | - | - | - | 5 | - | - |
| EQ4 | - | - | - | - | 3 | - | - | - | - | - | - | - |
| EQ5 | - | - | - | - | - | 3 | - | - | - | - | - | - |
| FSAA1 | 2 | 3 | - | - | - | 2 | 2 | - | - | - | - | - |
| FSAA2 | - | - | 1 | - | - | - | - | - | - | - | - | - |
| FSAA3 | - | - | - | 2 | - | - | - | - | - | - | - | - |
| FSAA4 | - | - | - | - | 2 | - | - | - | - | - | 5 | - |
| Cetearyl Alcohol | - | - | - | - | - | - | 4 | - | - | - | - | 5 |
| Amodimethicone | 20 | 20 | 20 | 20 | 20 | 20 | 20 | 20 | 20 | 20 | 20 | 20 |
| Climbazole | 1 | 1 | 1 | 1 | 1 | 1 | 1 | 1 | 1 | 1 | 1 | 1 |
| Wasser | ad 100 | | | | | | | | | | | |

| ***Stabilität*** | | | | | | | | | | | | |
|---|---|---|---|---|---|---|---|---|---|---|---|---|
| - nach 1 d, 20 °C | +++ | +++ | +++ | +++ | +++ | +++ | +++ | +++ | +++ | +++ | +++ | +++ |
| - nach 1 w, 20 °C | +++ | +++ | +++ | +++ | +++ | +++ | +++ | ++ | ++ | ++ | ++ | ++ |
| - nach 4 w, 20 °C | +++ | +++ | +++ | +++ | +++ | +++ | +++ | + | ++ | ++ | ++ | ++ |
| - nach 1 w, 40 °C | ++ | ++ | ++ | ++ | ++ | ++ | +++ | - | + | + | + | + |
| - nach 4 w, 40 °C | ++ | ++ | ++ | ++ | ++ | ++ | ++ | -- | -- | -- | - | - |

**Tabelle 2**

| **Beispiele für kosmetische Zubereitungen (Wasser, Konservierungsmittel ad 100 Gew.-%)** | | | | | | |
|---|---|---|---|---|---|---|
| **Zusammensetzung (INCI)** | **1** | **2** | **3** | **4** | **5** | **6** |
| **Dehyquart® A 04** Dioleoyl/adipinoylmethylethoxymonium Methosulfate | 2,0 | 2,0 | 2,0 | 2,0 | 4,0 | 4,0 |
| **Coco fatty acid N,N dimethylamino ethyl amide** | 0,6 | 0,6 | 0,6 | 0,6 | 0,6 | 0,6 |
| **Eumulgin® B2** Ceteareth-20 | 0,8 | 0,8 | - | 0,8 | - | 1,0 |
| **Eumulgin® VL 75** Lauryl Glucoside (and) Polyglyceryl-2 Polyhydroxystearate (and) Glycerin | - | - | 0,8 | - | 0.8 | - |
| **Lanette® O** Cetearyl Alcohol | 2,5 | 2,5 | 2,5 | 2,5 | 3,0 | 2,5 |
| **Cutina® GMS** Glyceryl Stearate | 0,5 | 0,5 | 0,5 | 0,5 | 0,5 | 1,0 |
| **Cetiol® HE** PEG-7 Glyceryl Cocoate | 1,0 | - | - | - | - | - |
| **Cetiol® PGL** Hexyldecanol (and) Hexyldecyl Laurate | - | 1,0 | - | - | 1,0 | - |
| **Cetiol® V** Decyl Oleate | - | - | - | 1,0 | - | - |
| **Eutanol® G** Octyldodecanol | - | - | 1,0 | - | - | 1,0 |
| **Nutrilan® Keratin W** Hydrolyzed Keratin | - | - | - | 2,0 | - | - |
| **Generol® 122 N** Soja Sterol | - | - | - | - | 1,0 | 1,0 |
| **Hydagen® CMF** Chitosan | 1,0 | 1,0 | 1,0 | 1,0 | 1,0 | 1,0 |
| **Copherol® 1250** Tocopherol Acetate | - | - | 0,1 | 0,1 | - | - |
| (1-4) Haarspülung, (5-6) Haarkur, | | | | | | |

## Patentansprüche

1. Verwendung von Mischungen, enthaltend
(a) Esterquats, deren Acylreste sich von Gemischen von Monocarbonsäuren und Dicarbonsäuren ableiten, und
(b) Fettsäureamidoamine
zur Herstellung von kosmetischen Zubereitungen.

2. Verwendung nach Anspruch 1, **dadurch gekennzeichnet, dass** man als Esterquats der Formel **(I)** einsetzt, 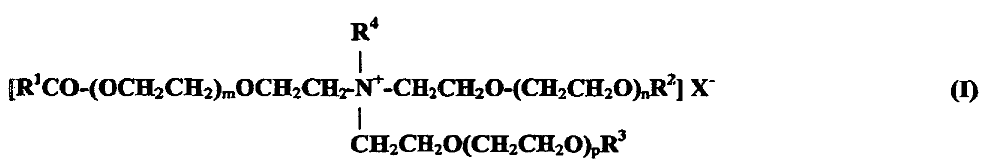 in der R¹CO für einen Monoacylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

3. Verwendung nach Anspruch 1, **dadurch gekennzeichnet, dass** man Esterquats der Formel **(II)** einsetzt, 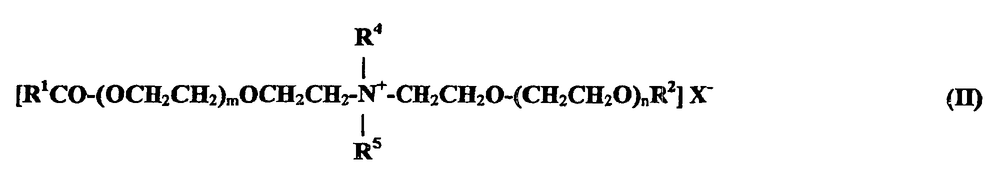 in der R¹CO für einen Monoacylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

4. Verwendung nach Anspruch 1, **dadurch gekennzeichnet, dass** man Esterquats der Formel **(III)** einsetzt, 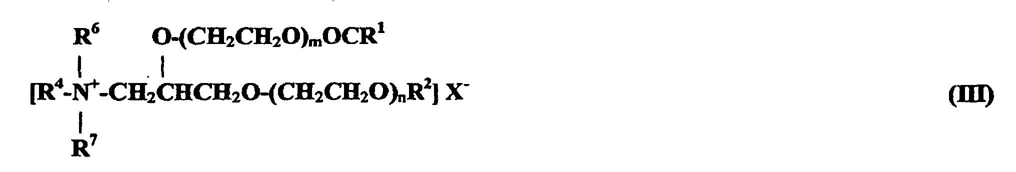 in der R¹CO für einen Monoacylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

5. Verwendung nach Anspruch 1, **dadurch gekennzeichnet, dass** man Esterquats der Formel **(IV)** einsetzt, 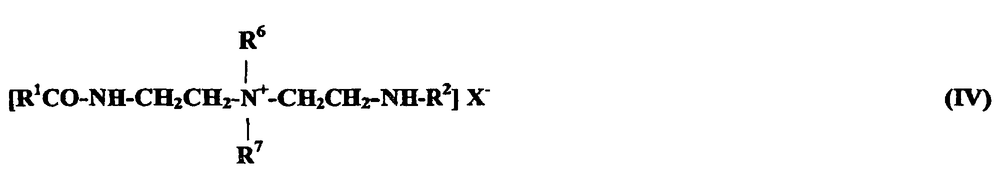 in der R¹CO für einen Monoacylrest mit 6 bis 22 Kohlenstoffatomen, R² für Wasserstoff oder R¹CO, R⁶ und R⁷ unabhängig voneinander für Alkylreste mit 1 bis 4 Kohlenstoffatomen und X für Halogenid, Alkylsulfat oder Alkylphosphat steht.

6. Verwendung nach Anspruch 1, **dadurch gekennzeichnet, dass** man Esterquats der Formel **(V)** einsetzt, 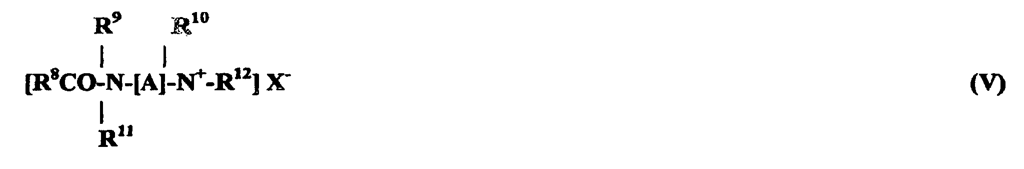 in der R⁸CO für einen gesättigten und/oder ungesättigten ethoxylierten Hydroxymonoacylrest mit 16 bis 22, vorzugsweise 18 Kohlenstoffatomen sowie 1 bis 50 Oxyethyleneinheiten, A für einen linearen oder verzweigten Alkylenrest mit 1 bis 6 Kohlenstoffatomen, R⁹, R¹⁰ und R¹¹ unabhängig voneinander für Wasserstoff oder eine Alkylgruppe mit 1 bis 4 Kohlenstoffatomen, R¹² für einen Alkylrest mit 1 bis 4 Kohlenstoffatomen oder einen Benzylrest und X für Halogen, Alkylsulfat oder Alkylphosphat steht.

7. Verwendung nach mindestens einem der Ansprüche 1 bis 6, **dadurch gekennzeichnet, dass** man Esterquats einsetzt, deren Monacylreste sich von C₈-C₁₀-Vorlauffettsäure, Talgfettsäure, Palmfettsäure, Kokosfettsäure, Ölsäure, Ricinolsäure sowie deren Härtungsprodukten und Gemischen ableiten.

8. Verwendung nach Anspruch 7, **dadurch gekennzeichnet, dass** man Esterquats einsetzt, deren Mono- und Diacylreste sich von Gemischen von Fettsäuren mit 12 bis 18 Kohlenstoffatomen und Adipinsäure ableiten.

9. Verwendung nach mindestens einem der Ansprüche 1 bis 8, **dadurch gekennzeichnet, dass** man als Komponente (b) Fettsäureamidoamine der Formel **(VI)** einsetzt,
**R**^{**13**}**CO-[NH(B)]**_{**n**}**NR**^{**14**}**R**^{**15**} **(VI)**
in der R¹³ für einen linearen oder verzweigten, gesättigten oder ungesättigten Acylrest mit 6 bis 22 Kohlenstoffatomen, R¹⁴ und R¹⁵ unabhängig voneinander für Wasserstoff, einen gegebenenfalls hydroxysubstituierten Alkylrest mit 1 bis 4 Kohlenstoffatomen, B für eine lineare oder verzweigte Alkylengruppe mit 2 bis 4 Kohlenstoffatomen und n für 1 oder 2 steht.

10. Verwendung nach mindestens einem der Ansprüche 1 bis 9, **dadurch gekennzeichnet, dass** man Fettsäureamidoamine einsetzt, deren Amidkomponente sich von Ethylendiamin, Propylendiamin, Diethylentriamin, Dipropylentriamin, Aminoethylethanolamin, N,N,-Dimethylaminopropylamin sowie deren Gemischen ableitet.

11. Verwendung nach mindestens einem der Ansprüche 1 bis 10, **dadurch gekennzeichnet, dass** man die Esterquats und die Fettsäureamidoamine im Gewichtsverhältnis 90 : 10 bis 10 : 90 einsetzt.

12. Verwendung nach mindestens einem der Ansprüche 1 bis 11, **dadurch gekennzeichnet, dass** man die Esterquats und die Fettsäureamidoamine gemeinsam mit Fettalkoholen und/oder Fettalkoholpolyglycolethern einsetzt.

13. Verwendung nach Anspruch 12, **dadurch gekennzeichnet, dass** man Fettalkohole bzw. Fettalkoholpolyglycolether der Formel **(VII)** einsetzt,
**R**^{**16**}**O(CH**_{**2**}**CH**_{**2**}**O)**_{**m**}**H (VII)**
in der R¹⁶ für einen linearen oder verzweigten Alkyl- und/oder Alkenylrest mit 6 bis 22 Kohlenstoffatomen und m für 0 oder Zahlen von 1 bis 20 steht.

14. Verwendung nach den Ansprüchen 12 und/oder 13, **dadurch gekennzeichnet, dass** man die Esterquats und Fettsäureamidoamine einerseits und die Fettalkohole bzw. Fettalkoholpolyglcolether andererseits im Gewichtsverhältnis 90 : 10 bis 10 : 90 einsetzt.

15. Verwendung nach mindestens einem der Ansprüche 1 bis 14, **dadurch gekennzeichnet, dass** man Haarpflegemittel herstellt.

16. Verwendung nach Anspruch 15, **dadurch gekennzeichnet, dass** man Haarkonditioniermittel herstellt.

17. Verwendung nach den Ansprüchen 15 und/oder 16, **dadurch gekennzeichnet, dass** man Haarkonditioniermittel in Emulsionsform herstellt.

18. Verwendung nach mindestens einem der Ansprüche 1 bis 14, **dadurch gekennzeichnet, dass** man Hautpflegemittel herstellt.

19. Verwendung nach Anspruch 18, **dadurch gekennzeichnet, dass** man Hautpflegemittel in Emulsionsform herstellt.

## Claims

1. The use of mixtures containing
(a) esterquats of which the acyl groups are derived from mixtures of monocarboxylic acids and dicarboxylic acids and
(b) fatty acid amidoamines
for the production of cosmetic preparations.

2. The use claimed in claim 1, **characterized in that** esterquats corresponding to formula **(I):** 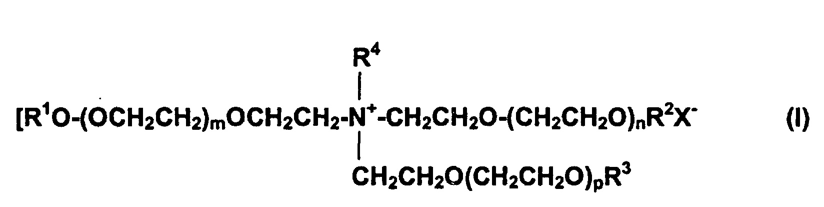 in which R¹CO is a monoacyl group containing 6 to 22 carbon atoms, R² and R³ independently of one another represent hydrogen or have the same meaning as R¹CO, R⁴ is an alkyl group containing 1 to 4 carbon atoms or a (CH₂CH₂O)_{q}H group, m, n and p together stand for 0 or numbers of 1 to 12, q is a number of 1 to 12 and X is halide, alkyl sulfate or alkyl phosphate, are used.

3. The use claimed in claim 1, **characterized in that** esterquats corresponding to formula **(II):** 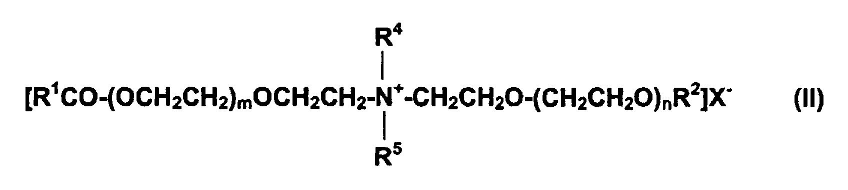 in which R¹CO is a monoacyl group containing 6 to 22 carbon atoms, R² is hydrogen or has the same meaning as R¹CO, R⁴ and R⁵ independently of one another are alkyl groups containing 1 to 4 carbon atoms, m and n together stand for 0 or numbers of 1 to 12 and X stands for halide, alkyl sulfate or alkyl phosphate, are used.

4. The use claimed in claim 1, **characterized in that** esterquats corresponding to formula **(III):** 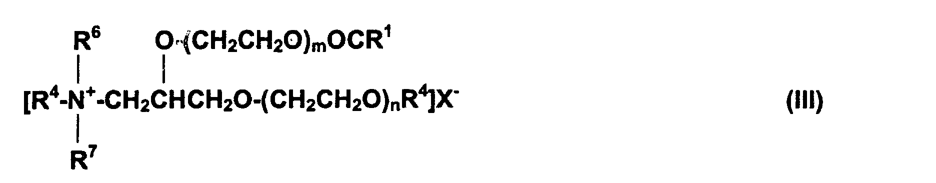 in which R¹CO is a monoacyl group containing 6 to 22 carbon atoms, R² is hydrogen or has the same meaning as R¹CO, R⁴, R⁶ and R⁷ independently of one another are alkyl groups containing 1 to 4 carbon atoms, m and n together stand for 0 or numbers of 1 to 12 and X stands for halide, alkyl sulfate or alkyl phosphate, are used.

5. The use claimed in claim 1, **characterized in that** esterquats corresponding to formula **(IV):** 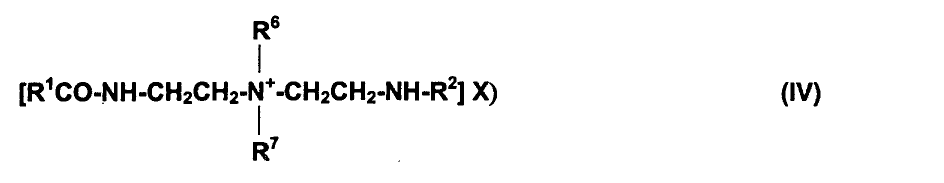 in which R¹CO is a monoacyl group containing 6 to 22 carbon atoms, R² is hydrogen or has the same meaning as R¹CO, R⁶ and R⁷ independently of one another are alkyl groups containing 1 to 4 carbon atoms and X is halide, alkyl sulfate or alkyl phosphate, are used.

6. The use claimed in claim 1, **characterized in that** esterquats corresponding to formula **(V):** 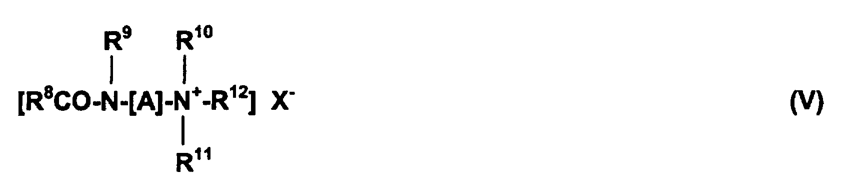 in which R⁸CO is a saturated and/or unsaturated ethoxylated hydroxymonoacyl group containing 16 to 22 and preferably 18 carbon atoms and 1 to 50 oxyethylene units, A is a linear or branched alkylene group containing 1 to 6 carbon atoms, R⁹, R¹⁰ and R¹¹ independently of one another represent hydrogen or a C₁₋₄ alkyl group, R¹² is a C₁₋₄ alkyl group or a benzyl group and X is halogen, alkyl sulfate or alkyl phosphate, are used.

7. The use claimed in at least one of claims 1 to 6, **characterized in that** esterquats of which the monoacyl groups are derived from C₈₋₁₀ head-fractionated fatty acid, tallow fatty acid, palm oil fatty acid, coconut oil fatty acid, oleic acid, ricinoleic acid and hydrogenation products and mixtures thereof are used.

8. The use claimed in at least one of claims 1 to 7, **characterized in that** esterquats of which the monoacyl and diacyl groups are derived from mixtures of C₁₂₋₁₈ fatty acids and adipic acid are used.

9. The use claimed in at least one of claims 1 to 8, **characterized in that** fatty acid amidoamines corresponding to formula **(VI):**
**R**^{**13**}**CO-[NH(B)]**_{**n**}**NR**^{**14**}**R**^{**15**} **(VI)**
in which R¹³ is a linear or branched, saturated or unsaturated acyl group containing 6 to 22 carbon atoms, R¹⁴ and R¹⁵ independently of one another represent hydrogen, an optionally hydroxysubstituted alkyl group containing 1 to 4 carbon atoms, B is a linear or branched alkylene group containing 2 to 4 carbon atoms and n is the number 1 or 2,
are used as component (b).

10. The use claimed in at least one of claims 1 to 9, **characterized in that** fatty acid amidoamines of which the amide component is derived from ethylenediamine, propylenediamine, diethylenetriamine, dipropylenetriamine, aminoethyl ethanolamine, N,N-dimethyl aminopropylamine and mixtures thereof are used.

11. The use claimed in at least one of claims 1 to 10, **characterized in that** the esterquats and the fatty acid amidoamines are used in a ratio by weight of 99:10 to 10:90.

12. The use claimed in at least one of claims 1 to 11, **characterized in that** the esterquats and the fatty acid amidoamines are used together with fatty alcohols and/or fatty alcohol polyglycol ethers.

13. The use claimed in claim 12, **characterized in that** fatty alcohols or fatty alcohol polylgycol ethers corresponding to formula **(VIII):**
**R**^{**16**}**O(CH**_{**2**}**CH**_{**2**}**O)**_{**m**}**H (VII)**
in which R¹⁶ is a linear or branched alkyl and/or alkenyl group containing 6 to 22 carbon atoms and m is 0 or a number of 1 to 20,
are used.

14. The use claimed in claims 12 and/or 13, **characterized in that** the esterquats and fatty acid amidoamines on the one hand and the fatty alcohols or fatty alcohol polyglycol ethers on the other hand are used in a ratio by weight of 90:10 to 10:90.

15. The use claimed in at least one of claims 1 to 14, **characterized in that** hair care preparations are produced.

16. The use claimed in claim 15, **characterized in that** hair conditioners are produced.

17. The use claimed in claims 15 and/or 16, **characterized in that** hair conditioners in the form of emulsions are produced.

18. The use claimed in at least one of claims 1 to 14, **characterized in that** skin care preparations are produced.

19. The use claimed in claim 18, **characterized in that** skin care preparations in the form of emulsions are produced.

## Revendications

1. Utilisation de mélanges contenant
(a) des esters d'ammonium quaternaire dont les radicaux acyles sont dérivés de mélanges d'acides monocarboxyliques et d'acides dicarboxyliques et
(b) des amidoamines d'acide gras
pour la fabrication de préparations cosmétiques.

2. Utilisation selon la revendication 1,
**caractérisée en ce qu'**
on utilise, des esters d'ammonium quaternaire de formule (I) 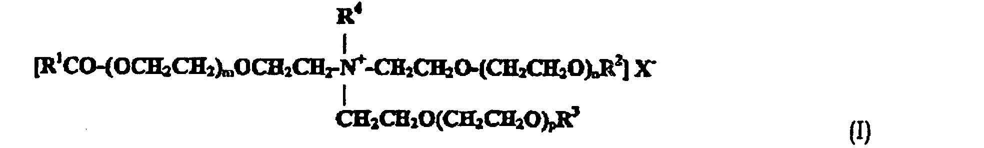 dans laquelle R¹CO représente un radical monoacyle portant 6 à 22 atomes de carbone, R² et R³, représentent indépendamment l'un de l'autre, un atome d'hydrogène ou R¹CO, R⁴ représente un radical alkyle portant 1 à 4 atomes de carbone ou un groupe (CH₂CH₂O)_{q}H, m, n et p sont égaux, au total à 0 ou à des nombres de 1 à 12, q représente un nombre de 1 à 12, et X est un halogénure, un sulfate d'alkyle ou un phosphate d'alkyle.

3. Utilisation selon la revendication 1,
**caractérisée en ce qu'**
on met en oeuvre des esters d'ammonium quaternaire de formule (II) 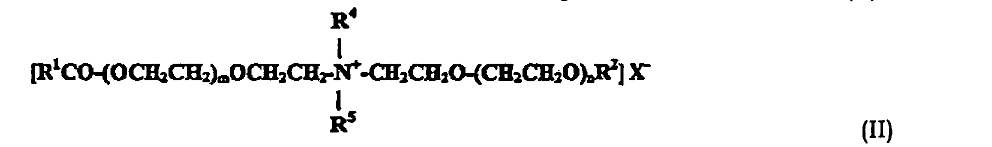 dans laquelle R¹CO représente un radical monoacyle portant 6 à 22 atomes de carbone, R² représente un atome d'hydrogène ou R¹CO, R⁴ et R⁵, représentent indépendamment l'un de l'autre, des radicaux alkyle portant 1 à 4 atomes de carbone, m et n sont égaux au total à 0 ou à des nombres de 1 à 12, et X est un halogénure, un sulfate d'alkyle ou un phosphate d'alkyle.

4. Utilisation selon la revendication 1,
**caractérisée en ce qu'**
on met en oeuvre des esters d'ammonium quaternaire de formule (III) 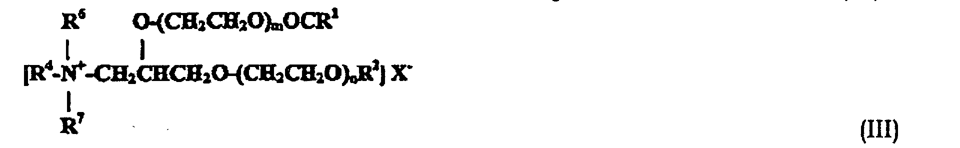 dans laquelle R¹CO représente un radical monoacyle portant 6 à 22 atomes de carbone, R² représente un atome d'hydrogène ou R¹CO, R4 R6 et R⁷, représentent indépendamment les uns aux autres, des radicaux alkyle portant 1 à 4 atomes de carbone, m et n sont égaux au total à 0 ou à des nombres de 1 à 12, et X est un halogénure, un sulfate d'alkyle ou un phosphate d'alkyle.

5. Utilisation selon la revendication 1,
**caractérisée en ce qu'**
on met en oeuvre des esters d'ammonium quaternaire de formule (IV) 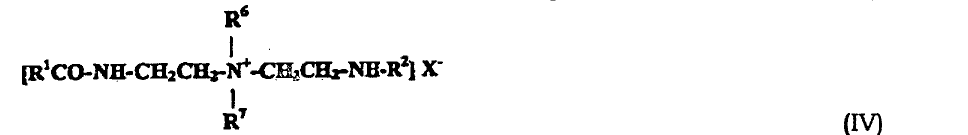 dans laquelle R¹CO représente un radical monoacyle portant 6 à 22 atomes de carbone, R² représente un atome d'hydrogène ou R¹CO, R⁶ et R⁷, représentent indépendamment l'un de l'autre, des radicaux alkyle portant 1 à 4 atomes de carbone, et X est un halogénure, un sulfate d'alkyle ou un phosphate d'alkyle.

6. Utilisation selon la revendication 1,
**caractérisée en ce qu'**
on met en oeuvre des esters d'ammonium quaternaire de formule **(V)** 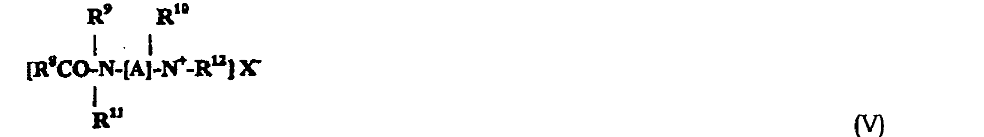 dans laquelle R⁸CO représente un radical hydroxy monoacyle éthoxylé saturé et/ou insaturé, portant 16 à 22 atomes de carbone, de préférence 18 atomes de carbone ainsi qu'une à 50 unités d'oxyéthylène, A représente un radical alkylène linéaire ou ramifié portant 1 à 6 atomes de carbone, R⁹, R¹⁰ et R¹¹ représentent indépendamment les uns des autres, un atome d'hydrogène ou un groupe alkyle portant 1 à 4 atomes de carbone, R¹² représente un radical alkyle portant 1 à 4 atomes de carbone ou un radical benzyle, et X est un halogénure, un sulfate d'alkyle ou un phosphate d'alkyle.

7. Utilisation selon au moins l'une des revendications 1 à 6,
**caractérisée en ce qu'**
on met en oeuvre des esters d'ammonium quaternaire d'ammonium quaternaire dont les radicaux monoacyles sont dérivés d'acide gras précurseur en C₈ à C₁₀, d'acide gras de suif, d'acide gras de palme, d'acide gras de coco, d'acide oléique, d'acide ricinolique ainsi que de leurs produits de l'hydrogénation et des mélanges.

8. Utilisation selon la revendication 7,
**caractérisée en ce qu'**
on met en oeuvre des esters d'ammonium quaternaire dont les radicaux mono et diacyles sont dérivés de mélanges d'acides gras portants 12 à 18 atomes de carbone et d'acide adipique.

9. Utilisation selon au moins l'une des revendications 1 à 8,
**caractérisée en ce qu'**
on met en oeuvre en tant que composant (b) des amidoamines d'acide gras de formule (VI)
R¹³CO-[NH(B)]ₙNR¹⁴R¹⁵ (VI)
dans laquelle R¹³ représente un radical acyle linéaire ou ramifié, saturé ou insaturé portant 6 à 22 atomes de carbone, R¹⁴ et R¹⁵, représentent indépendamment l'un de l'autre, un atome d'hydrogène ou un radical acyle éventuellement substitué par un groupe hydroxy et portant 1 à 4 atomes de carbone, B représente un groupe alkylène linéaire ou ramifié portant 2 à 4 atomes de carbone, et n représente 1 ou 2.

10. Utilisation selon au moins l'une des revendications 1 à 9,
**caractérisée en ce qu'**
on met en oeuvre des amidoamines d'acide gras dont le composant amide est dérivé de l'éthylènediamine, de la propylènediamine, de la diéthylènetriamine, de la dipropylènetriamine, de l'aminoéthyléthanolamine, de la N,N-diméthylaminopropylamine ainsi que de leurs mélanges.

11. Utilisation selon au moins l'une des revendications 1 à 10,
**caractérisée en ce qu'**
on met en oeuvre les esters d'ammonium quaternaire et les amidoamines d'acide gras dans le rapport pondéral de 90: 10 à 10:90.

12. Utilisation selon au moins l'une quelconque des revendications 1 à 11,
**caractérisée en ce qu'**
on met en oeuvre les esters d'ammonium quaternaire et les amidoamines d'acide gras conjointement avec des alcools gras et/ou des polyglycoéthers d'alcool gras.

13. Utilisation selon la revendication 12,
**caractérisée en ce qu'**
on met en oeuvre des alcools gras ou des polyglycoléthers d'alcool gras de formule (VII)
R¹⁶O(CH₂CH₂O)ₘH (VII)
dans laquelle R¹⁶ représente un radical alkyle et/ou alcényle linéaire ou ramifié portant 6 à 22 atomes de carbone et m représente 0 ou des nombres de 1 à 20.

14. Utilisation selon les revendications 12 et/ou 13,
**caractérisée en ce qu'**
on met en oeuvre les esters d'ammonium quaternaire et les amidoamines d'acide gras d'une part, et les alcools gras ou les polyglycoléthers d'alcool gras d'autre part dans un rapport pondéral de 90: 10 à 10:90.

15. Utilisation selon au moins l'une des revendications 1 à 14,
**caractérisée en ce qu'**
on fabrique des agents de soin pour les cheveux.

16. Utilisation selon la revendication 15,
**caractérisée en ce qu'**
on fabrique des agents de conditionnement pour les cheveux.

17. Utilisation selon les revendications 15 et/ou 16,
**caractérisée en ce qu'**
on fabrique des agents de conditionnement pour les cheveux sous forme d'émulsion.

18. Utilisation selon au moins l'une des revendications 1 à 14,
**caractérisée en ce qu'**
on fabrique des agents de soin pour la peau.

19. Utilisation selon la revendication 18,
**caractérisée en ce qu'**
on fabrique dés agents de soin pour la peau sous forme d'émulsion.
